(19) Europäisches Patentamt
European Patent Office
Office européen des brevets

(11) Veröffentlichungsnummer: **0 019 713**
**B1**

(12) # EUROPÄISCHE PATENTSCHRIFT

(45) Veröffentlichungstag der Patentschrift: **16.02.83**

(21) Anmeldenummer: **80101996.9**

(22) Anmeldetag: **14.04.80**

(51) Int. Cl.³: **C 07 C 69/734**, C 07 C 69/757, C 07 C 67/00, C 07 C 67/30// C07D307/32, C07D303/20, C07D317/52, C07C47/277, C07C43/225, C07D317/60, C07D407/06, C07F9/40

(54) **Verfahren zur Herstellung von Styrylcyclopropancarbonsäureestern, neue Zwischenprodukte dafür und deren Herstellung.**

(30) Priorität: **23.04.79 DE 2916321**

(43) Veröffentlichungstag der Anmeldung: **10.12.80 Patentblatt 80/25**

(45) Bekanntmachung des Hinweises auf die Patenterteilung: **16.02.83 Patentblatt 83/07**

(84) Benannte Vertragsstaaten: **BE CH DE FR GB IT LI NL**

(56) Entgegenhaltungen:
**AT B 283 320**
**DE A 2 539 895**
**DE A 2 706 184**
**DE A 2 738 150**
**DE A 2 827 101**
**GB A 813 539**

(73) Patentinhaber: **BAYER AG**
**Zentralbereich Patente, Marken und Lizenzen**
**D-5090 Leverkusen 1, Bayerwerk (DE)**

(72) Erfinder: **Jautelat, Manfred, Dr.**
**Müllersbaum 28**
**D-5093 Burscheid (DE)**
Erfinder: **Arlt, Dieter, Dr.**
**Rybniker Strasse 2**
**D-5000 Köln 80 (DE)**
Erfinder: **Lantzsch, Reinhard, Dr.**
**Heymannstrasse 32**
**D-5090 Leverkusen (DE)**
Erfinder: **Fuchs, Rainer, Dr.**
**Roeberstrasse 8**
**D-5600 Wuppertal (DE)**
Erfinder: **Riebel, Hans-Jochem, Dr.**
**In der Beek 92**
**D-5600 Wuppertal (DE)**
Erfinder: **Schröder, Rolf, Dr.**
**Pahlkestrasse 17**
**D-5600 Wuppertal 1 (DE)**
Erfinder: **Harnisch, Horst, Dr.**
**Heinenbusch 4**
**D-5203 Much (DE)**

**EP 0 019 713 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von Styryl-cyclopropan-carbonsäureestern, neue Zwischenprodukte dafür und deren Herstellung

Die vorliegende Erfindung betrifft ein neues Verfahren zur Herstellung von Styryl-cyclopropancarbonsäureestern und Zwischenprodukte zur Durchführung dieser Verfahren und deren Herstellung.

Es ist bereits bekannt geworden, daß bestimmte Ester von 3-Styryl-2,2-dimethylcyclopropancarbonsäuren insektizide Eigenschaften besitzen (Deutsche Offenlegungsschrift 2 738 150). Es ist jedoch noch keine wirtschaftliche Synthese für Carbonsäuren dieser Art in technischem Maßstab bekannt.

Die Knüpfung der C-C-Doppelbindung der Styrylgruppe erfolgt durch eine Wittigreaktion bei der Butyllithium als Base verwendet wird und unter Schutzgas bei − 78 °C gearbeitet werden muß. Dieser Syntheseweg ist somit für eine technische Herstellung nicht gangbar.

Ferner ist auch keine für eine Herstellung im größeren Maßstab verwendbare Synthese für den 2,2-Dimethyl-3-formyl-1-carbonsäureester bekannt.

Gegenstand der vorliegenden Erfindung sind :

1. Verbindungen der allgemeinen Formel (II)

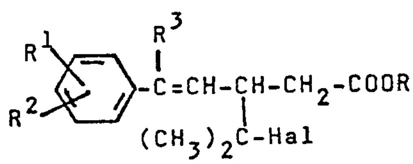

(II)

in welcher

$R^1$ für Alkoxy, Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht und

$R^3$ für Wasserstoff oder Chlor steht und

R für $C_{1-4}$-Alkyl steht und

Hal für Chlor oder Brom steht ;

2. Ein Verfahren zur Herstellung von Verbindungen der allgemeinen Formel (II)

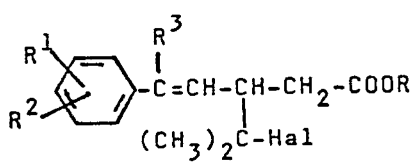

(II)

in welcher

$R^1$ für Alkoxy, Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht und

$R^3$ für Wasserstoff oder Chlor steht und

R für $C_{1-4}$-Alkyl steht und

Hal für Chlor oder Brom steht, dadurch gekennzeichnet, daß Verbindungen der allgemeinen Formel (III)

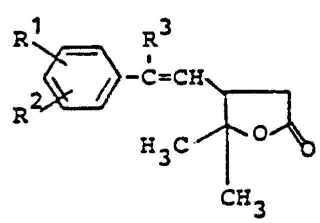

(III)

in welcher

$R^1$, $R^2$ und $R^3$ die oben angegebene Bedeutung haben, mit einem Halogenierungsmittel sowie gleichzeitig oder nacheinander mit einem Alkohol der Formel (IV)

$$R—OH \quad (IV)$$

in welcher

R die oben angegebene Bedeutung hat, gegebenenfalls in Gegenwart eines Verdünnungsmittels und gegebenenfalls in Gegenwart von gasförmigem Halogenwasserstoff, umgesetzt werden ;

3. Die Verwendung der Verbindungen der Formel (II) zur Herstellung von Styryl-cyclopropan-1-carbonsäureester der allgemeinen Formel (I)

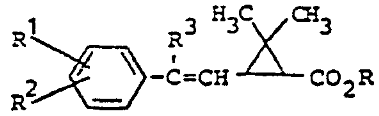

(I)

in welcher

R für $C_{1-4}$-Alkyl steht,

$R^1$ für Alkoxy, Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht und

$R^3$ für Wasserstoff oder Chlor steht, dadurch gekennzeichnet, daß man Verbindungen der allgemeinen Formel (II)

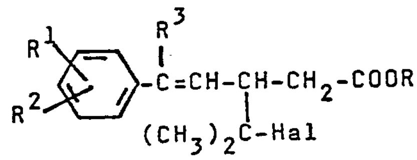

(II)

in welcher

R, $R^1$, $R^2$, $R^3$ die oben angegebene Bedeutung besitzen und

Hal für Chlor oder Brom steht, mit einer gegebenenfalls wässrigen Base gegebenenfalls in gegenwart eines Verdünnungsmittels in Gegenwart eines Phasentransferkatalysators umsetzt ; sowie

4. Das Verfahren gemäß 3. (oben), das dadurch gekennzeichnet ist, daß nicht zu starke Basen verwendet werden und in Gegenwart eines Phasentransferkatalysators gearbeitet wird.

5. Die Verbindungen der Formel III, in welcher $R^1$, $R^2$ und $R^3$ die unter 1. (oben) angegebene Bedeutung haben, sind neu.

6. Die neuen Verbindungen der Formel (III) werden erhalten, indem man

a) Verbindungen der Formel V

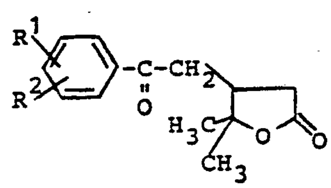

(V)

in welcher

$R^1$ und $R^2$ die oben angegebene Bedeutung haben, mit Phosphorpentachlorid umsetzt oder

b) Verbindungen der Formel (VI)

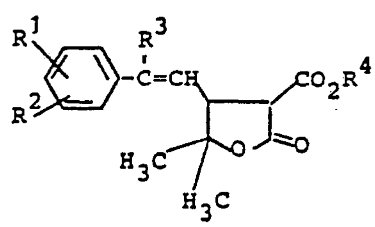

(VI)

in welcher

$R^1$, $R^2$ und $R^3$ die unter 1. (oben) angegebene Bedeutung haben und

$R^4$ $C_{1-4}$-Alkyl bedeutet, decarboxyliert oder verseift und decarboxyliert oder

c) Verbindungen der Formel (V) in welcher $R^1$ und $R^2$ die oben angegebene Bedeutung haben reduziert und Wasser abspaltet.

7. Die Verbindungen der Formel (V), in welcher $R^1$ und $R^2$ die unter 1. (oben) angegebene Bedeutung haben, sind neu.

8. Die Verbindungen der Formel (VI), in der $R^1$, $R^2$, $R^3$ und $R^4$ die unter 6. (oben) angegeben Bedeutung haben, sind neu.

9. Verbindungen der allgemeinen Formel (VI) werden erhalten, indem man Verbindungen der allgemeinen Formel VII

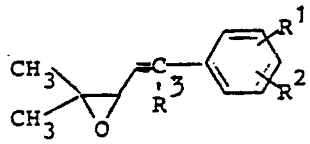

(VII)

in welcher

$R^1$, $R^2$ und $R^3$ die unter 1. (oben) angegebene Bedeutung haben mit Malonestern der Formel VIII

$$CH_2(CO_2R^4)_2 \qquad (VIII)$$

in welcher

$R^4$ $C_{1-4}$-Alkyl bedeutet, in Gegenwart einer Base sowie gegebenenfalls in Gegenwart eines Verdünnungdmittels umsetzt.

10. Es wurde ferner gefunden, daß die Verbindungen der Formel (VI) bevorzugt nach Verfahren 9 (oben) dann erhalten werden, wenn man unterhalb von 50 °C arbeitet.

11. Die Verbindungen der Formel (VII), in welcher $R^1$, $R^2$ und $R^3$ die unter 1. (oben) angegebene Bedeutung besitzen, sind neu.

12. Die Verbindungen der Formel (VII) werden erhalten, indem man Verbindungen der Formel (IX)

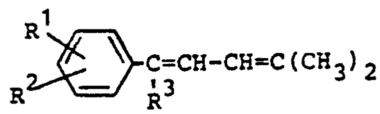

(IX)

in welcher

$R^1$, $R^2$, $R^3$ die oben angegebene Bedeutung haben, oxidiert.

13. Die Verbindungen der Formel (IX), in welcher $R^1$, $R^2$ und $R^3$ die unter 1. (oben) angegebene Bedeutung haben, sind neu.

14. Die Verbindungen der Formel (IX) werden erhalten, indem man

a) Verbindungen der Formel (XA) oder (XB)

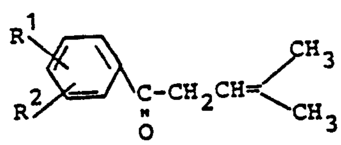

(XA)

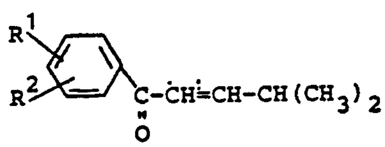

(XB)

in welcher

$R^1$ und $R^2$ die unter 1. (oben) angegebene Bedeutung haben, oder ein Gemisch derselben, mit Phosphorpentachlorid und anschließend einer Base, umsetzt oder

b) Verbindungen der Formel (XI)

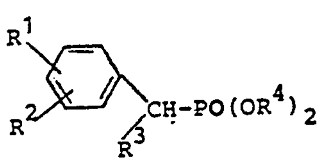

(XI)

in welcher

$R^1$, $R^2$, $R^3$ und $R^4$ die unter 6. (oben) angegebene Bedeutung haben, mit Dimethylacrolein der Formel (XII)

$$(CH_3)_2C = CH—CHO \quad (XII)$$

umsetzt oder

c) Verbindungen der Formel (XVII)

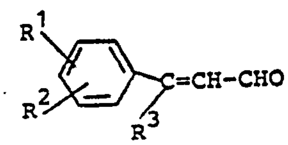

(XVII)

in welcher

$R^1$, $R^2$ und $R^3$ die unter 1. (oben) angegebene Bedeutung haben, mit der Verbindung der Formel (XVIII)

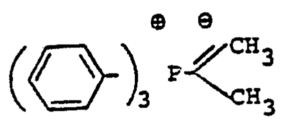

(XVIII)

umsetzt oder

d) Verbindungen der Formel (XVII) mit Isopropylmagnesiumchlorid der Formel (XIX)

$$(i—C_3H_7—MgCl) \quad (XIX)$$

umsetzt.

15. Verbindungen der allgemeinen Formel (XVII) werden erhalten,

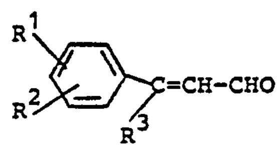

(XVII)

in welcher

$R^1$, $R^2$ die unter 1. (oben) angegebene Bedeutung haben und

$R^3$ für Chlor steht, indem man Acetophenone der allgemeinen Formel (XVI)

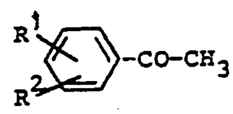

(XVI)

in welcher

$R^1$ und $R^2$ die unter 1. (oben) angegebene Bedeutung haben, ohne Zusatz eines Verdünnungsmittels mit $POCl_3$ und Dimethylformamid umsetzt und die Reaktionsprodukte nach Hydrolyse ohne destillative Aufarbeitung isoliert.

16. Bestimmte Verbindungen der Formel (V)

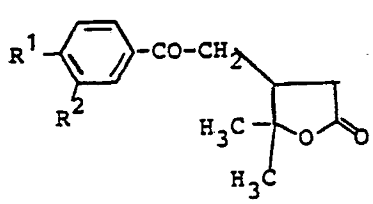

(V)

in welcher

$R^1$ und $R^2$ die oben angegebene Bedeutung haben, jedoch nicht durch Halogen substituiert sein

dürfen, werden erhalten, indem man die Verbindung der Formel (XX)

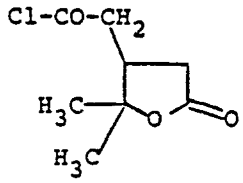

(XX)

mit Verbindungen der (XXI)

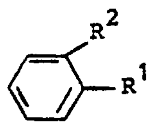

(XXI)

in welcher

$R^1$ und $R^2$ die unter 16. angegebene Bedeutung haben in Gegenwart eines Friedel-Crafts-Katalysators und gegebenenfalls in Gegenwart eines Verdünnungsmittels umsetzt.

17. Die Verbindung der Formel (XX) wird erhalten, indem man die Verbindung der Formel (XXII)

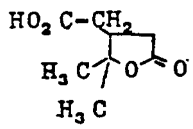

(XXII)

mit einem Chlorierungsmittel umsetzt.

Die nach dem erfindungsgemäßen Verfahren 3 (oben) herstellbaren Styrylcyclopropancarbonsäure ester der Formel (I) dienen zur Herstellung insektizider und akarizider Wirkstoffe.

Verwendet man beim Verfahren 3 (oben) 5-(4'-Methoxy-phenyl)-5-chlor-3-(1'-chlor-1'-methyl)-ethyl-pent-4-ensäureethylester als Ausgangstoff, kann der Reaktionsablauf durch folgendes Formelschema wiedergegeben werden :

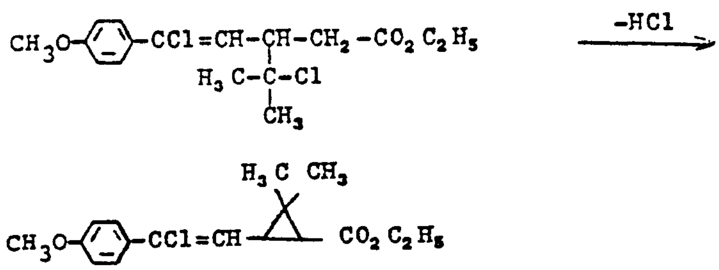


Die beim Verfahren 3 verwendbaren Ausgangsstoffe sind durch die allgemeinen Formel (II) definiert, in welcher bevorzugt.

$R^1$ für $C_1$-$C_4$-Alkoxy, $C_1$-$C_4$-Alkylthio, $C_1$-$C_2$-Fluor-alkoxy, $C_1$-$C_2$-Chlorfluoralkoxy oder $C_1$-$C_2$-Fluoralkylthio steht,

$R^2$ für Wasserstoff, Methoxy oder zusammen mit $R^1$ für $C_1$-$C_2$-Alkylendioxy oder $C_1$-$C_2$-Fluoralkylendioxy steht,

$R^3$ für Chlor steht und

R $C_{1-4}$-Alkyl steht.

Verfahren 3 wird durchgeführt, indem man die Verbindungen der Formel (II) gegebenenfalls in einem Verdünnungsmittel löst, den Katalysator hinzufügt und anschließend die Base hinzugibt. Als Verdünnungsmittel kommen beispielsweise Kohlenwasserstoffe wie Benzin, Petrolether, Benzol, Toluol, Xylol, Cyclohexan, Chlorkohlenwasserstoffe wie Methylenchlorid, Chlorbenzol, Dichlorbenzol oder Nitrile, wie Acetonitril in Frage.

Ganz allgemeinen können alle inerten, nicht mit Wasser mischbaren Verdünnungsmittel verwendet werden.

Als Basen werden bevorzugt wäßrige Basen wie NaOH oder KOH verwendet, wobei der Wassergehalt sehr gering sein kann. So ist es beispielsweise auch möglich technisches, pulverisates Kaliumhydroxyd ohne weiteren Wasserzusatz zu verwenden. Ferner können auch Natrium- oder Kaliumcarbonat, insbesondere unter Verwendung von Acetonitril als Base verwendet werden.

Das Verfahren wird bevorzugt in Gegenwart eines Phasentransferkatalysators durchgeführt.

Als Phasentransferkatalysator kommen beispielsweise Kronenether, Tetraalkylphosphoniumsalze oder bebevorzugt Tetraalkylammoniumsalze in Frage. Als ganz besonders bevorzugte Katalysatoren seien genannt :

Tetrapropylammoniumbromid, Tetrabutylammoniumchlorid und -bromid, Benzyltriethylammoniumchlorid, Methyltrioctylammoniumchlorid.

Die Katalysatormenge kann in weiten Grenzen schwanken. Im allgemeinen haben sich 0,1-15 Gew.-%, vorzugsweise 0,3 bis 10 Gew.-%, bezogen auf das Gewicht der eingesetzten Verbindungen der allgemeinen Formel (II) bewährt.

Das Verfahren wird bei Temperaturen zwischen – 20 °C und 80 °C, bevorzugt zwischen 0 und 50 °C, durchgeführt.

Bei höheren Temperaturen wird ein zusätzliches Molekül Chlorwasserstoff abgespalten und man erhält nicht die gewünschten Verbindungen der allgemeinen Formel (I).

Es wird bevorzugt unter Normaldruck gearbeitet.

Die Verwendung von billigen wäßrigen Basen ist ein entscheidender technischer Fortschritt, da ähnliche Reaktionen sonst nur mit starken, wasserfreien Basen durchgeführt werden können, wie beispielsweise Natriumhydrid, Kalium- tert.-Butylat, Natrium- tert.-Amyltat (US-P 3 652 652 ; US-P 3 077 496). Natriumhydrid ist im technischen Maßstab nur schwierig zu handbaren ; zudem liefern solche Basen bei ähnlichen Reaktionen nur unbefriedigende Ausbeuten.

Die Ergebnisse sind jedoch in den Fällen in denen $R^3$ für Chlor steht noch schlechter, da sich mit solch starken Basen aus der β-Chlor-β-aryl-vinyl-Gruppe, bedingt durch die Arylgruppe, sehr leicht Chlorwasserstoff abspalten läßt.

Aufgabe des erfindungsgemäßen Verfahrens war es daher, Basen zu finden, die eine solche Abspaltung unter bestimmten Bedingungen nicht bewirken. Dies ist überraschenderweise mit den erfindungsgemäßen Basen und bei den erfindungsgemäßen Reaktionstemperaturen nicht der Fall. Zudem ist es als äußerst überraschend anzusehen, daß auch bei Verwendung wäßriger Basen praktisch keine Verseifung der Estergruppe auftritt, die ja bekanntlich im alkalischen Medium unter sehr milden Bedingungen verläuft.

Nach Verfahren 3. lassen sich bevorzugt folgende Cyclopropancarbonsäureester der Formel (I) herstellen :

2,2-Dimethyl-3-[2'-chlor-2'-(4'-methoxy-phenyl)-vinyl]-cyclopropan-1-carbonsäuremethyl und -ethylester

2,2-Dimethyl-3-[2'-chlor-2'-(4'-ethoxy-phenyl)-vinyl]-cyclopropan-1-carbonsäuremethyl und -ethylester

2,2-Dimethyl-3-[2'-chlor-2'-(3',4'-dimethoxy-phenyl)-vinyl]-cyclopropan-1-carbonsäuremethyl- und -ethylester

2,2-Dimethyl-3-[2'-chlor-2'-(4'-trifluormethoxy-phenyl)-vinyl]-cyclopropan-1-carbonsäuremethyl- und -ethylester

2,2-Dimethyl-3-[2'-chlor-2'-(4'-chlordifluormethoxy-phenyl)-vinyl]-cyclopropan-1-carbonsäuremethyl- und -ethylester

2,2-Dimethyl-3-[2'-chlor-2'-(3'-methoxy-phenyl)-vinyl]-cyclopropan-1-carbonsäuremethyl und -ethylester

2,2-Dimethyl-3-[2'-chlor-2'-(3',4'-methylendioxy-phenyl)-vinyl]-cyclopropan-carbonsäuremethyl- und ethylester

2,2-Dimethyl-3-[2'-chlor-2'-(4'-trifluormethylthio-phenyl)-vinyl]-cyclopropan-1-carbonsäuremethyl- und -ethylester

2,2-Dimethyl-3-[2'-chlor-2'-(3'-trifluormethoxy-phenyl)-vinyl]-cyclopropan-1-carbonsäuremethyl- und -ethylester

2,2-Dimethyl-3-[2'-chlor-2'-(3',4'-difluormethylendioxy-phenyl)-vinyl]-cyclopropan-1-carbonsäuremethyl- und ethylester

2,2-Dimethyl-3-[2'-chlor-2'-(3',4'-trifluorethylendioxy-phenyl)-vinyl]-cyclopropan-1-carbonsäuremethyl- und -ethylester

2,2-Dimethyl-3-[2'-chlor-2'-(1,1,2,2-tetrafluor-ethoxy-phenyl)-vinyl]-cyclopropan-1-carbonsäuremethyl- und ethylester

2,2-Dimethyl-3-[2'-chlor-2'-(4'-trifluormethylmercapto-phenyl)-vinyl]-cyclopropan-1-carbonsäuremethyl- und ethylester.

Die Verbindungen der allgemeinen Formel (II) sind neu. Sie werden nach dem unter 2 (oben) angegebenen Verfahren erhalten, indem man Verbindungen der allgemeinen Formel (III) mit einem Halogenierungsmittel sowie gleichzeitig oder nacheinander mit einem Alkohol der Formel (IV) gegebenenfalls in Gegenwart eines Verdünnungsmittels und gegebenenfalls in Gegenwart vongasförmigen Halogenwasserstoff umsetzt.

Verwendet man bei Verfahren 2. 4,4-Dimethyl-3-[2'-(4'-methoxy-phenyl)-2'-(chlor)-vinyl]-γ-butyrolacton, Thionylchlorid und Ethanol als Ausgangsstoffe, kann der Reaktionsverlauf durch folgendes Formelschema wiedergegeben werden :

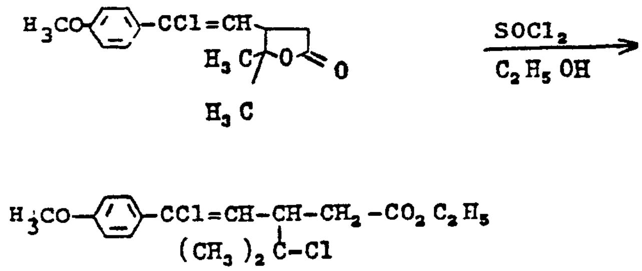


Die beim Verfahren 2 (oben) verwendbaren Ausgangsstoffe sind durch die allgemeine Formel (III) definiert. Die bevorzugten Substituenten $R^1$, $R^2$ und $R^3$ sind die gleichen wie beim Verfahren 3. Die Verbindungen der Formel (III) sind neu ; ihre Herstellung wird weiter unten beschrieben.

Folgende Halogenierungsmittel können bei Verfahren 2. Verwendung finden : Chlorwasserstoff, Bromwasserstoff, Thionylchlorid, Phosgen, Phosphortrichlorid, Phosphorpentachlorid, bevorzugt ist Thionylchlorid.

Bevorzugte Alkohole, die bei Verfahren 2. Verwendung finden können sind :

Methanol, Ethanol, Propanol, iso-Propanol, Butanol, 3-Phenoxy-benzylalkohol, 3-Phenoxy-α-cyano-benzylalkohol, 3-Phenoxy-4-fluor-α-cyano-benzylalkohol.

Ganz besonders bevorzugt sind Methanol und Ethanol.

Als Verdünnungsmittel kommen beispielsweise in Frage : Benzol, Toluol, Benzin, Petrolether, Cyclohexan, Chlorbenzol, Xylol, Chloroform.

Die Reaktionstemperatur liegt zwischen 30 und 150 °C, vorzugsweise zwischen 50 und 100 °C.

Die Durchführung des Verfahrens kann auf zwei grundsätzlich verschiedene Arten erfolgen.

Entweder wird der Ausgangstoff der allgemeinen Formel (III) in einem Alkohol der Formel (IV) gelöst, gegebenenfalls noch ein Verdünnungsmittel zugesetzt und anschließend das Halogenierungsmittel eingeleitet oder zugetropft.

Bevorzugt wird jedoch zunächst ohne Alkoholzusatz der Ausgangsstoff der allgemeinen Formel (III) mit dem Halogenierungsmittel, gegebenenfalls unter Druck und gegebenenfalls in Gegenwart eines Verdünnungsmittels erhitzt. Anschließend wird der Alkohol der allgemeinen Formel (IV), gegebenenfalls in Gegenwart zusätzlichen Chlorwasserstoffs zugetropft oder zugepumpt. Das Halogenierungsmittel wird bevorzugt im Überschuß eingesetzt.

Die Isolierung der Verbindungen der allgemeinen Formel (II) erfolgt durch Abdestillieren des Lösungsmittels. Eine weitere Reinigung ist schwierig, aber auch nicht notwendig. Die rohen Verbindungen der Formel (II) können direkt für Verfahren 3 verwendet werden.

Die Verbindungen der allgemeinen Formel (III) sind neu. Sie werden nach dem unter 6. (oben) angegebenen Verfahren erhalten, indem man

a) Verbindungen der Formel (V) mit Phosphorpentachlorid umsetzt oder

b) Verbindungen der Formel (VI) decarbalkoxyliert oder zur Carbonsäure verseift und decarboxyliert oder

c) Verbindungen der Formel (V) reduziert und anschließend Wasser abspaltet.

Verwendet man bei Verfahren 6a (oben) 4,4-Dimethyl-3-(4'-trifluormethoxy-phenacyl)-γ-butyrolacton als Ausgangsstoff, so kann der Reaktionsverlauf durch folgendes Formelschema wiedergegeben werden :

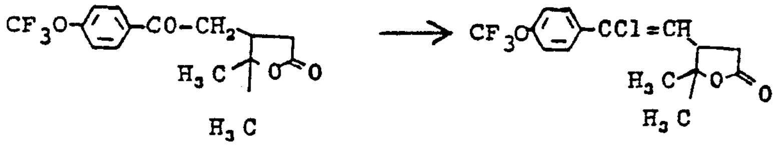

Die beim Verfahren 6a (oben) verwendbaren Ausgangsstoffe sind durch die allgemeine Formel (V) definiert. Die bevorzugten Substituenten $R^1$ und $R^2$ sind die gleichen wie bei Verfahren 3.

Die Verbindungen der Formel (V) sind neu.

Ihre Herstellung erfolgt analog der Herstellung von 4,4-Dimethyl-3-phenacyl-γ-butyrolacton nach dem in J. Org. Chemistry, Band 39, No 17, Seite 2604 (1974) angegebenen Verfahren.

Diese Verbindungen können jedoch auch nach dem Verfahren 16 (oben) erhalten werden.

Als Chlorierungsmittel kann Phosphorpentachlorid verwendet werden.

Verfahren 6a (oben) wird in der Weise durchgeführt, daß man die Ausgangstoffe der Formel (V) mit 1 bis 2,5 Äquivalenten des Chlorierungsmittels, vorzugsweise 1,1 bis 2 Äquivalenten, umsetzt.

Vorzugsweise wird — im Gegensatz zur allgemein üblichen Arbeitsweise (Houben-Weyl, Band V, 3 Seite 912 ff) bei der Umsetzung von Ketonen mit Phosphorpentachlorid — in Gegenwart eines Verdünnungsmittels gearbeitet.

Als Verdünnungsmittel kommen insbesondere Kohlenwasserstoffe wie Benzin, Petrolether, Cyclohexan, Benzol, Toluol, Chlorbenzol in Frage, aber auch Chlorkohlenwasserstoffe wie Methylenchlorid, Chloroform, Tetrachlorkohlenstoff, Dichlorethan oder Chlorbenzol, sowie Nitrile, wie Acetonitril.

Die Umsetzungstemperatur liegt zwischen – 20 °C und + 60 °C, vorzugsweise zwischen 0 °C und 35 °C.

Die Aufarbeitung erfolgt durch Rühren des Reaktionsansatzes mit Wasser, abtrennen der organischen Phase, sowie Abdestillieren des Lösungsmittels. Die Reinigung der Verbindung der allgemeinen Formel (III) erfolgt durch Destillation oder durch Umkristallisieren.

Verwendet man bei Verfahren 6b (oben) 4,4-Dimethyl-3-[2-(4'-trifluor-methoxy-phenyl)-2'-(chlor)-vinyl]-2-ethoxycarbonyl-γ-butyrolacton als Ausgangsstoff, kann der Reaktionsverlauf durch folgendes Formelschema wiedergegeben werden :

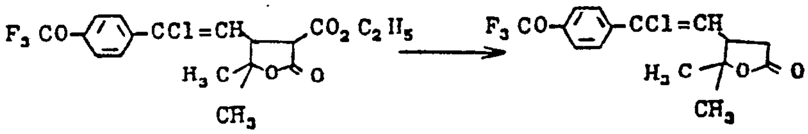

Die beim Verfahren 6b (oben) verwendbaren Ausgangsstoffe sind durch die allgemeinen Formel (VI) definiert. Die bevorzugten Substituenten $R^1$, $R^2$ und $R^3$ sind die gleichen wie bei Verfahren 3, $R^4$ steht bevorzugt für Methyl oder Ethyl.

Die Verbindungen der Formel (VI) sind neu ; ihre Herstellung wird weiter unten beschrieben.

Die Entfernung einer Alkoxycarbonylgruppe kann grundsätzlich im neutralen, sauren oder alkalischen Medium erfolgen.

Im erfindungsgemäßen Verfahren 6b erfolgt dies bevorzugt im neutralen oder im sauren Medium.

Hierzu werden die Verbindungen der allgemeinen Formel (VI) auf Temperaturen zwischen 80 und 180 °C, bevorzugt auf Temperaturen zwischen 100 und 150 °C, vorzugsweise in einem Verdünnungsmittel und gegebenenfalls in Gegenwart einer Säure bzw. eines sauren Katalysators erhitzt.

Als Verdünnungsmittel seien beispielsweise genannt : Wasser, Dimethylsulfoxid oder phosphorhaltige Lösungsmittel, wie sie in der DT-OS 2 638 453 beschrieben sind. Ferner kommen aber auch alle anderen inerten Lösungsmittel mit genügend hohem Siedepunkt in Betracht. Bei der Verwendung von Dimethylsulfoxid oder phosphorhaltiger Lösungsmittel wird bevorzugt unter Verwendung von Wasser und Salzen gearbeitet. Verwendet man ausschließlich Wasser als Verdünnungsmittel, so muß unter Druck gearbeitet werden und die bevorzugte Temperatur liegt oberhalb 120 °C.

Bevorzugt ist jedoch das Arbeiten in Gegenwart einer Säure bzw. eines sauren Katalysators. Als solche seien beispielsweise genannt :

Schwefelsäure, Phosphorsäure, Sulfonsäuren oder saure Ionenaustauscher. Hierbei wird entweder unter Druck gearbeitet oder aber vorzugsweise der gebildete Alkohol $R^4$—OH laufend aus der Mischung, gegebenenfalls zusammen mit dem verwendeten Lösungsmittel, abdestilliert.

Die Isolierung der Verbindung der Formel (III) erfolgt in üblicher Weise durch Abfiltrieren oder Extrahieren.

Die Verbindungen der allgemeinen Formel (VI) sind neu. Sie werden nach dem unter 9 (oben) angegebenen Verfahren erhalten, indem man Verbindungen der allgemeinen Formel (VII) mit Malonestern der Formel (VIII) in Gegenwart einer Base sowie gegebenenfalls in Gegenwart eines Verdünnungsmittels umsetzt.

Verwendet man bei Verfahren 6c (oben) 4,4-Dimethyl-3-(4'-methoxy-phenacyl)-γ-butyrolacton als Ausgangsstoff, so kann der Reaktionsverlauf durch folgendes Formelschema wiedergegeben werden :

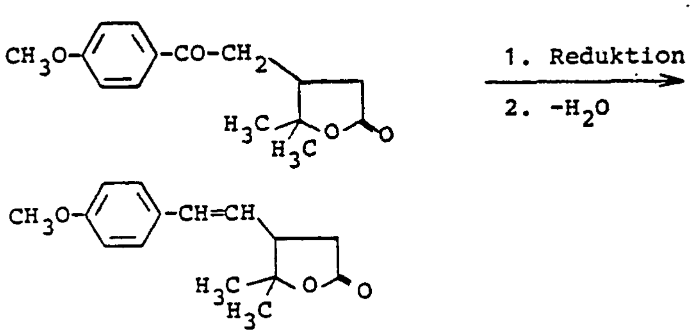


Die beim Verfahren 6c verwendbaren Ausgangsstoffe sind durch die allgemeine Formel (V) definiert. Die bevorzugten und besonders bevorzugten Substituenten $R^1$ und $R^2$ sind die gleichen wie bei Verfahren 3.

Erster Teilschritt des Verfahrens 6c ist eine Reduktion.

Als Reduktionsmittel kommen grundsätzlich alle Mittel in Frage, durch die ein Keton zum Alkohol reduziert wird, ohne den Lactonring anzugreifen. Beispielsweise seien genannt : Komplexe Borhydride, wie Natrium- oder Kaliumborhydrid oder Wasserstoff in Gegenwart von Beispielsweise Nickel-, Palladium-, oder Platin-Katalysatoren, wie beispielsweise Raney-Nickel. Die Verwendung von Natriumborhydrid ist bevorzugt.

Zweiter Teilschritt des Verfahrens 6c ist eine Dehydratisierung.

Zur Dehydratisierung werden bevorzugt saure Katalysatoren eingesetzt. Beispielsweise seien genannt : Oxalsäure, Schwefelsäure, Phosphorsäure, Kaliumhydrogensulfat, p-Toluolsulfonsäure, Aluminiumoxid, Silikate.

Außerdem kann der entstandene Alkohol acetyliert und anschließend durch Erhitzen Essigsäure abgespalten werden. Die Acetylierung erfolgt durch Acetylchlorid oder Acetanhydrid.

Verwendet man bei Verfahren 9 (oben) 2,2-Dimethyl-3-[2'-(4'-ethoxy-phenyl)-2'-(chlor)-vinyl]-oxiran und Malonsäuredimethylester als Ausgangstoffe, so kann der Reaktionsverlauf durch folgendes Formelschema wiedergegeben werden :

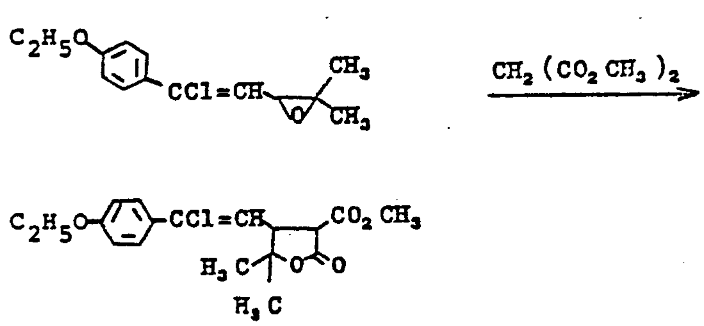


Die beim Verfahren 9 (oben) verwendbaren Ausgangsstoffe sind durch die allgemeinen Formeln (VII) und (VIII) definiert. Die Verbindungen der Formel (VIII) sind bekannt, die bevorzugten Substituenten $R^4$ sind Methyl und Ethyl. Die Verbindungen der allgemeinen Formel (VII) sind neu. Die bevorzugten Substituenten $R^1$, $R^2$ und $R^3$ sind die gleichen wie bei Verfahren 3.

Ihre Herstellung wird weiter unten beschrieben.

Verfahren 9 wird durchgeführt, indem man zunächst das Natrium- oder Kaliumsalz des Malonesters der Formel (VIII) herstellt, was üblicherweise mit Alkoholaten, wie Natriummethylat oder Natriumethylat durchgeführt wird. Als Verdünnungsmittel werden bevorzugt Alkohole, wie Methanol oder Ethanol, verwendet.

Zwar ist die Reaktion gemäß Verfahren 9 prinzipiell bekannt, jedoch nicht mit den verwendeten erfindungsgemäßen Substituenten. Es mußten daher Verfahrensbedingungen gefunden werden, unter denen die gegen Alkali empfindliche Aryl-chlor-vinyl-Gruppe nicht angegriffen wird.

Es wurde gefunden, daß die Verbindungen der Formel (VI) bevorzugt dann erhalten werden, wenn man unterhalb von 50 °C arbeitet. Die bevorzugte Reaktionstemperatur liegt somit bei Verfahren 9 zwischen 0 und 50 °C, besonders bevorzugt zwischen 20 und 40 °C.

Das Oxiran der allgemeinen Formel (VII) wird bei dieser Temperatur zur Suspension des Malonestersalzes in einem Verdünnungsmittel zugetropft und die Reaktionslösung noch einige Zeit, üblicherweise etwa 1-6 Stunden bei der gewählten Reaktionstemperatur gehalten.

Durch Aufarbeitung in üblicher Weise kann man die Verbindung der Formel (VI) isolieren oder aber direkt nach Verfahren 6b weiter umsetzen.

Die Verbindungen der allgemeinen Formel (VII) sind neu. Sie werden nach dem unter 12 (oben) angegebenen Verfahren erhalten, indem man Verbindungen der Formel (IX) oxidiert.

Verwendet man bei Verfahren 12 (oben) 1,1-Dimethyl-4-(4'-trifluor-methylthio-phenyl)-4-chlor-1,3-butadien als Ausgangsstoff, so kann der Reaktionsverlauf durch folgendes Formelschema wiedergegeben werden :

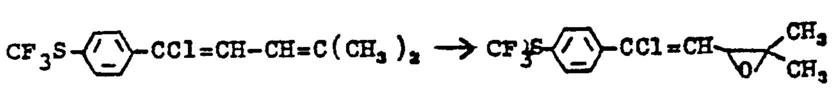

Die beim Verfahren 12 verwendbaren Ausgangsstoffe sind durch die allgemeine Formel (IX) definiert. Die bevorzugten Substituenten sind die gleichen wie bei Verfahren 3.

Die Verbindungen der Formel (IX) sind neu ; ihre Herstellung wird weiter unten beschrieben.

Die überführung einer Doppelbindung in ein Oxiran ist grundsätzlich bekannt, jedoch liefert die Epoxidierung von Dienen meist nur schlechte Ausbeuten, da grundsätzlich beide Doppelbindungen angegriffen werden können.

Ferner führt die Verwendung von Persäuren sehr oft zu Folgereaktionen, da unter sauren Bedingungen Oxirane wieder geöffnet werden können.

Aufgabe des erfindungsgemäßen Verfahrens war es daher, Oxidationsmittel zu finden, die in guter Ausbeute und Selektivität zu dem gewünschten Oxiran der allgemeinen Formel (VII) führen. Überraschenderweise wurde gefunden, daß mit aromatischen Persäuren im Gegensatz zu aliphatischen Persäuren in guter Ausbeute und Selektivität die gewünschten Oxirane entstehen. Das ist insofern überraschend, da aromatische Persäuren starke Oxidationsmittel darstellen und man annehmen mußte, daß mit diesen beiden Doppelbindungen angegriffen werden, wie dies mit aliphatischen Persäuren, wie beispielsweise Perpropionsäure der Fall ist. Als Lösungsmittel für die Oxidation kommen Benzol, Methylenchlorid oder Chloroform in Frage. Gegebenenfalls kann in Gegenwart von Natriumcarbonat gearbeitet werden. Die Reaktionstemperatur liegt zwischen 0 und 50 °C, vorzugsweise bei Raumtemperatur.

Grundsätzlich können die gewünschten Oxirane auch über die Chlorhydrine mit Natriumhypochlorit und anschließende Chlorwasserstoffabspaltung erhalten werden. Doch liefert dieses Verfahren etwas schelchtere Ausbeuten als die Oxidation mit Persäuren und außerdem werden zwei statt einer Stufe benötigt.

Die Verbindungen der allgemeinen Formel (IX) sind neu. Sie werden nach dem unter 14 (oben) angegebenen Verfahren erhalten, indem man

a) Verbindungen der Formel (XA) oder (XB) oder ein Gemisch derselben mit Phosphorpentachlorid und anschließend einer Base umsetzt oder

b) Verbindungen der Formel (XI) mit Dimethylacrolein der Formel (XII) umsetzt oder

c) Verbindungen der Formel (XVII) mit Verbindungen der Formel (XVIII) umsetzt oder

d) Verbindungen der Formel (XVII) mit Isopropylmagnesiumchlorid umsetzt.

Verwendet man bei Verfahren 14a ein Gemisch aus 2-Methyl-5-oxo-5-(4′-methoxy)-phenyl-2-penten und 2-Methyl-5-oxo-5-(4′-methoxy)-phenyl-3-penten als Ausgangsstoff, so kann der Reaktionsverlauf durch folgendes Formelschema wiedergegeben werden :

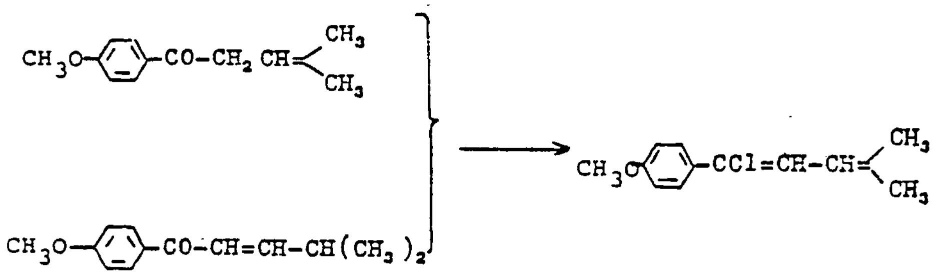

Die beim Verfahren 14a verwendbaren Ausgangsstoffe sind durch die allgemeine Formel (XA) bzw. (XB) definiert. Die bevorzugten Substituenten $R^1$ und $R^2$ sind die gleichen wie bei Verfahren 3.

Die Verbindungen der Formel (X) sind neu. Ihre Herstellung erfolgt analog zu dem in J. Org. Chemistry Band 25, Seite 272 beschriebenen Verfahren. Durch Kondensation der Acetophenone der Formel (XVI) mit Isobutyraldehyd erhält man dimere Produkte, die durch Destillation in Gegenwart von Natriumacetat in ein Monomerengemisch der Formel (XA und B) gespalten werden :

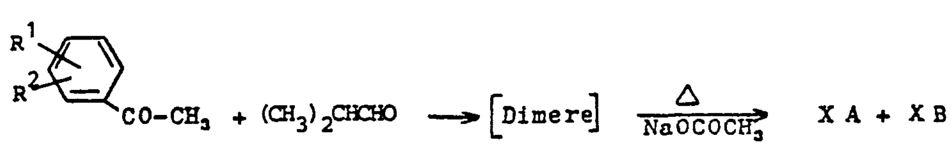


(XVI)

Als Beispiele für die Acetophenone der Formel (XVI) seien genannt : 4-Ethoxyacetophenon, 4-Methylmercaptoacetophenon, 4-Methoxyacetophenon, 3,4-Dioxymethylenacetophenon.

Verfahren 14a wird erfindungsgemäß in der Weise durchgeführt, daß die Ausgangsstoffe der allgemeinen Formel (XA bzw. XB bzw. Gemische von beiden in einem Verdünnungsmittel vorgelegt werden und $PCl_5$ hinzudosiert wird.

Im Gegensatz zur allgemein üblichen Arbeitsweise bei der Umsetzung von Ketonen mit Phosphorpentachlorid (Houben-Weyl, Band V, 3, Seite 912 ff) wird Verfahren 14a bevorzugt in Gegenwart eines Verdünnungsmittels durchgeführt. Als Verdünnungsmittel kommen beispielsweise Kohlenwasserstoffe, wie Benzin, Petrolether, Pentan, Hexan, Cyclohexan, Benzol, Toluol, Xylol oder Chlorkohlenwasserstoffe wie Methylenchlorid, Tetrachlorkohlenstoff, Dichlorethan oder Chlorbenzol, sowie Nitrile, wie Acetonitril in Frage. Bevorzugt ist Toluol, Cyclohexan und Tetrachlorkohlenstoff.

Die Umsetzung eines ungesättigten Ketons mit $PCl_5$ ist praktisch immer problematisch und gibt Anlaß zu einer Vielzahl von Nebenreaktionen.

So kann beispielsweise $PCl_5$ eine Chlorierung in Allylstellung bzw. in α-Stellung zur Carbonylgruppe

bewirken. Der hierbei sowie durch Eliminierung entstehende Chlorwasserstoff kann sich wieder an eine Doppelbindung addieren. Außerdem vermag $PCl_5$ auch Doppelbindungen zu chlorieren, so daß höherchlorierte Produkte entstehen.

Es wurde gefunden, daß sich diese Nebenreaktionen praktisch vollständig vermeiden lassen, wenn man in Gegenwart von Verdünnungsmitteln und bei relativ tierfer Temperatur arbeitet sowie anschließend bei der gleichen oder bei einer niedrigeren Temperatur eine Base zusetzt, so daß das Vorhandensein von Chlorwasserstoff bei höheren Temperaturen vermieden wird.

Verfahren 14a wird bei Temperaturen zwischen – 40 °C und + 30 °C durchgeführt, bevorzugt sind Temperaturen zwischen – 10 °C und + 20 °C.

Als Basen kommen Alkoholate, wie Natriummethylat oder Natriumethylat in Frage. Besonders bevorzugt sind wäßrige Basen, wie Natriumhydroxid, Kaliumhydroxid, Natriumcarbonat, Kaliumcarbonat in Gegenwart von Phasentransferkatalysatoren.

Als Phasentransferkatalysatoren werden bevorzugt die gleichen eingesetzt wie bei Verfahren 3.

Die Base wird in ca. der vierfachen äquimolaren Menge eingesetzt, da sie mit dem Phosphoroxichlorid reagiert und außerdem zur Chlorwasserstoffeliminierung dient.

Die Diene der allgemeinen Formel (IX) können durch Destillation gereinigt werden. Falls das Dien nicht unzersetzt destillierbar ist, wird es durch sogenanntes « Andestillieren », d. h. durch längeres Erhitzen unter vermindertem Druck auf mäßig erhöhte Temperaturen, gereinigt.

Verwendet man bei Verfahren 14b (oben) 4-Trifluormethylmercapto-α-chlorbenzyl-O,O-diethyl-phosphonsäureester und Dimethylacrolein als Ausgangsstoffe, so kann der Reaktionsverlauf durch folgendes Formelschema wiedergegeben werden :

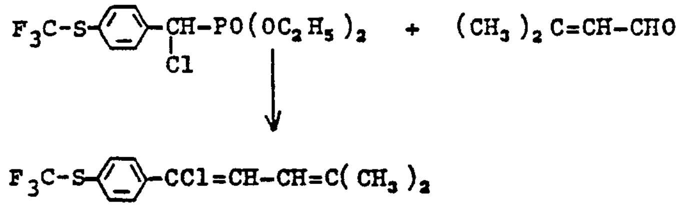

Die beim Verfahren 14b verwendbaren Ausgangsstoffe sind durch die Formeln (XI) und (XII) definiert. Dimethylacrolein (XII) ist literaturbekannt.

Die α-Chloro-benzyl-phosphonsäureester der Formel (XI) sind teilweise bekannt. Sie können durch Umsetzung von α-Hydroxy-benzyl-phosphonsäureestern der Formel (XIII)

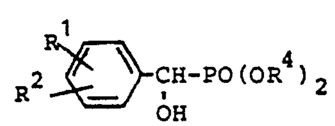 (XIII)

in welcher

$R^1$, $R^2$ und $R^4$ die unter 14 (oben) angegebene Bedeutung haben, mit einem Chlorierungsmittel, wie z. B. Thionylchlorid oder Phosphoroxichlorid, bei Temperaturen zwischen 0 und 100 °C, vorzugsweise bei 20 bis 80 °C, hergestellt werden (vergleiche Chimia *28* (1974), 656-657 ; J. Am. Chem. Soc. 87 (1965), 2 777-2 778).

Die zur Herstellung der Ausgangsverbindungen der Formel (XII) zu verwendenden α-Hydroxy-benzyl-phosphonsäureester sind durch Formel (XIII) definiert.

Die α-Hydroxy-benzyl-phosphonsäureester der Formel (XIII) sind teilweise bekannt. Man erhält sie nach bekannten Verfahren, im allgemeinen durch Umsetzung von Aldehyden der Formel

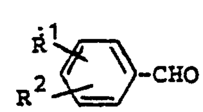 (XIV)

worin

$R^1$ und $R^2$ die oben angegebene Bedeutung haben, mit Phosphorigsäureester der Formel

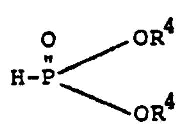 (XV)

worin

$R^4$ die oben angegebene Bedeutung hat, gegebenenfalls in Gegenwart eines Katalysators wie z. B. Triethylamin, bei Temperaturen zwischen 0 und 150 °C, vorzugsweise bei 20 bis 100 °C (vergleiche

Houben-Weyl, Methoden der organischen Chemie, 4. Auflage (1963), Band 12/1, S. 475-483, Georg Thieme Verlag, Stuttgart).

Als Beispiele für die Aldehyde der Formel (XIV) seien genannt : 4-Methoxybenzaldehyd, 4-Trifluormethoxybenzaldehyd, 4-Trifluormethylmercaptobenzaldehyd, 5-Formyl-2,2-difluor-1,3-benzodioxol, 5-Formyl-1,3-benzodioxol (Piperonal).

Als Beispiele der Phosphorigsäureester der Formel (XV) seien genannt :
Phosphorigsäuredimethylester, Phosphorigsäurediethylester.

Verfahren 14b ist eine Variante der Wittig-Reaktion und im Prinzip bekannt.

So erhält man beispielsweise bei der Umsetzung von Aldehyden mit α-Chloro-benzylphosphonsäureestern in Gegenwart einer Base Chloro-styrylverbindungen (Chima *28*, S. 656-657 ; 1974 und JACS *87*, S. 2777-2778 ; 1965).

Als Base wird hierzu nach dem Stand der Technik Natriumhydrid verwendet, welches ein teures und schwierig zu handhabendes Reagenz ist. Die Anwendung dieser Synthesemethode ist bisher nur auf wenige Beispiele beschränkt.

Dagegen kann nach dem erfindungsgemäßen Verfahren wesentlich kostengünstiger gearbeitet werden. Es ist einfacher und auch ohne weiteres in technischem Maßstab durchführbar.

Als weitere Vorteile des erfindungsgemäßen Verfahrens sind beispielsweise die Möglichkeit der Durchführung bei Raumtemperatur und in wasserhaltigen Reaktionsmedien, die Möglichkeit anstelle von Natriumhydrid relativ billige Basen zu verwenden, die vergleichsweise einfache Aufarbeitung und die guten Ausbeuten zu nennen.

Das Verfahren 14b zur Herstellung von Verbindungen der Formel (IX) wird im allgemeinen unter Verwendung von Verdünnungsmitteln durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan. Alkohole wie Methanol, Ethanol, n- und iso-Propanol, n-, iso-, sek.- und tert.- Butanol, sek.- und tert.- Butanol sowie Dimethylsulfoxid.

Bei Arbeiten im Zweiphasenmedium werden neben 50 %iger wäßriger Natronlauge mit Wasser praktisch nicht mischbare Solventien wie z. B. Benzin, Benzol oder Toluol verwendet.

Als Basen können die bei Carbonyl-Olefinierungen üblichen Basen verwendet werden. Genannt seien Alkalihydroxide wie z. B. Natrium-, Kaliumhydroxid, Alkalialkoholate wie z. B. Natrium- und Kaliummethylat, -ethylat, -n- und -isopropylat, -n-, -iso-, sek.- und tert.-Butylat, Alkalihydride wie z. B. Natriumhydrid sowie Alkallithiumverbindungen wie z. B. Butyllithium.

Vorzugsweise werden als Basen Natrium- und Kaliumhydroxid sowie Natriummethylat und -ethylat verwendet.

Die Reaktionstemperaturen liegen im allgemeinen zwischen − 70 °C und + 150 °C, vorzugsweise zwischen − 10 und + 50 °C. Die Umsetzung wird im allgemeinen bei Normaldruck durchgeführt.

Zur Durchführung des erfindungsgemäßen Verfahrens werden die Komponenten gewöhnlich in äquimolaren Mengen eingesetzt. Lediglich die Basen werden gewöhnlich in größerem Überschuß eingesetzt : bei Arbeiten im einphasigen System bis zu 30 Mol-%, vorzugsweise bis zu 15 Mol-% ; bei Verwendung von 50 %iger Natronlauge als zweiter Phase im allgemeinen das 5 bis 15-fache der stöchiometrisch erforderlichen Menge.

Alkoholate werden gegebenenfalls *in situ* aus den Alkoholen und Alkalimetallen hergestellt.

Im allgemeinen wird die Base und gegebenenfalls der Katalysator in einem oder mehreren der genannten Verdünnungsmittel vorgelegt. Dazu werden, gegebenenfalls in einem der angegebenen Solventien gelöst, die Reaktionskomponenten — α-Chloro-benzyl-phosphonsäureester und Aldehyd — in der angegebenen Reihenfolge zugegeben. Zur Vervollständigung der Reaktion wird die Mischung unter Rühren mehrere Stunden im angegebenen Temperaturbereich gehalten.

Zur Aufarbeitung versetzt man die Reaktionsmischung mit Wasser, säuert gegebenenfalls mit Salzsäure an und extrahiert mit Methylenchlorid. Die organische Phase wird getrocknet und das Lösungsmittel wird im Vakuum abdestilliert. Das als Rückstand verbleibende Produkt wird gegebenenfalls durch Vakuumdestillation, oder falls es nicht unzersetzt destillierbar ist, durch sogenanntes « Andestillieren », d. h. durch längeres Erhitzen unter vermindertem Druck auf mäßig erhöhte Temperaturen, gereinigt. Zur Charakterisierung dient der Siedepunkt oder der Brechungsindex.

Verwendet man bei Verfahren 14c β-(3,4-Methylendioxyphenyl)-β-chlor-acrolein und Triphenylisopropylphosphoran als Ausgangsstoffe, so kann der Reaktionsverlauf durch folgendes Formelschema wiedergegeben werden :

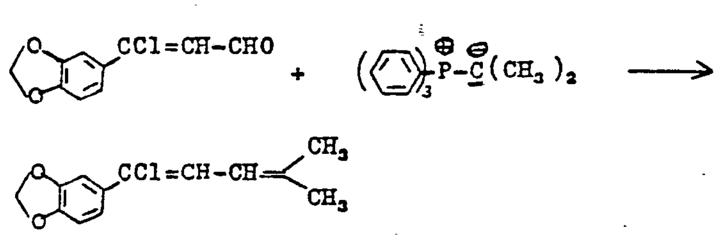

Die Herstellung des Phosphorans der Formel (XVIII) ist bekannt. Die Aldehyde der allgemeinen Formel (XVII) sind teilweise bekannt. Ihre Herstellung nach einem neuen Verfahren wird weiter unten beschrieben.

Die bei Verfahren 14c verwendbaren Ausgangstoffe sind durch die allgemeine Formel (XVII) definiert. Die bevorzugten Substituenten sind die gleichen wie bei Verfahren 3.

Als Beispiel für die Aldehyde der Formel (XVII) seien genannt : β-(4-Methoxyphenyl)-β-chlor-acrolein, β-(3,4-Methylendioxyphenyl)-β-chlor-acrolein.

Verfahren 14c ist eine Wittigreaktion und erfolgt nach im Prinzip bekannten Methoden.

Verwendet man bei Verfahren 14d β-(4-Methoxy-phenyl)-β-chloracrolein und Isopropylmagnesiumchlorid als Ausgangsstoffe, so kann der Reaktionsverlauf durch folgendes Formelschema wiedergegeben werden :

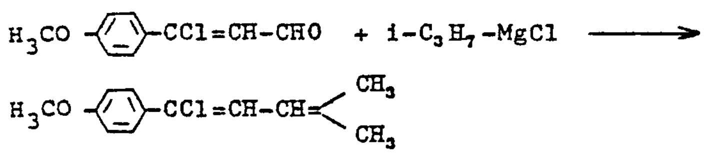

Die Herstellung von Isopropylmagnesiumchlorid (XIX) ist bekannt : Chem. Ber. 69 (1936), S. 1766.

Die Aldehyde der allgemeinen Formel (XVII) sind teilweise bekannt. Ihre Herstellung nach einem neuen Verfahren wird weiter unten beschrieben. Die bei Verfahren 14d verwendeten Ausgangsstoffe sind durch die allgemeine Formel (XVII) definiert. Die bevorzugten und besonders bevorzugten Substituenten sind die gleichen wie bei Verfahren 3.

Als Beispiele für die Aldehyde der Formel (XVII) seien genannt : β-(4-Methoxy-phenyl)-β-chlor-acrolein, β-(3,4-Methylendioxy-phenyl)-p-chlor-acrolein.

Verfahren 14d ist eine Grignard-Reaktion mit nachfolgender Dehydratisierung. Die Grignard-Reaktion erfolgt nach im Prinzip bekannter Weise. Es ist überraschend, daß aus dem intermediär gebildeten sekundären Alkohol verhältnismäßig leicht Wasser abgespalten werden kann.

Die Dehydratisierung erfolgt mit Säuren, vorzugsweise mit Schwefelsäure bei Temperaturen zwischen 0 und 50 °C, vorzugsweise bei 20° bis 45 °C. Die Isolierung der Diene erfolgt durch Eingießen in Wasser und Extrahieren in üblicher Weise.

Verwendet man bei Verfahren 15 (oben) 4-Methoxy-acetophenon als Ausgangsstoff, so kann der Reaktionsverlauf durch folgendes Formelschema wiedergegeben werden :

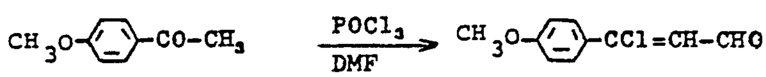


Die beim Verfahren 15 (oben) verwendbaren Ausgangsstoffe sind durch die allgemeine Formel (XVI)

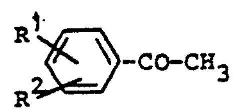
(XVI)

definiert. Die bevorzugten Substituenten sind die gleichen wie bei Verfahren 3.

Als Beispiele für Acetophenone der Formel (XVI) seien genannt : 4-Ethoxy-acetophenon, 4-Methoxy-acetophenon, 4-Methoxyacetophenon, 3,4-Dioxymethylenacetophenon.

Es wurde gefunden, daß man nach dem erfindungsgemäßen Verfahren 15 die β-Chloracroleine der allgemeinen Formel (XVII) in besonders einfacher Weise und mit besserer Ausbeute und Reinheit erhält, wenn man auf den in der Literatur (Proc. Chem. Soc. (London), 1958, 227 ; Angew. Chem. *71* ((1959), 573 ; Chem. Ber. *93* (1960), 2746 ; Chem. Ber. *98* (1965), 3554)) empfohlenen Zusatz eines Verdünnungsmittels und die verlustreiche destillative Aufarbeitung verzichtet und die Substanz nach schonender Hydrolyse bei tiefer Temperatur kristallin isoliert.

Man geht beim erfindungsgemäßen Verfahren in der Weise vor, daß man das Acetophenon der allgemeinen Formel (XVI) in Dimethylformamid löst und bei 15-100 °C, vorzugsweise bei 25-60 °C tropfenweise mit Phosphoroxychlorid versetzt und noch bei der gleichen Temperatur 1-5 Stunden nachrührt.

Anschließend wird auf Eiswasser gegeben und mit Natronlauge ein pH-Wert von ca. 5 eingestellt. Die Temperatur soll dabei nicht über 25 °C steigen. Nach einigem Nachrühren wird abgesaugt, mit Wasser gewaschen und im Vakuum getrocknet.

Verwendet man bei Verfahren 16 Anisol und 4,4-Dimethyl-3-chlor-carbonylmethyl-, γ-butyrolacton als Ausgangsstoffe, kann der Reaktionsablauf durch folgendes Formelschema wiedergegeben werden :

Die beim Verfahren 16 verwendbaren Ausgangsstoffe sind durch die Formeln (XX) und (XXI) definiert. Die Verbindung (XX) ist neu ; ihre Herstellung wird weiter unten beschrieben.

Die Verbindungen der Formel (XXI) sind bekannt, beispielsweise seien genannt :

Anisol, Ethoxybenzol, Benzodioxol, Brenzkatechindimethylether. Als Katalysatoren kommen grundsätzlich alle üblichen Friedel-Crafts-Katalysatoren in Frage, wie Aluminiumchlorid, Zinntetrachlorid, Titantetrachlorid, Fluorwasserstoff, Bortrifluorid, Eisen (III) chlorid, Zinkchlorid, Polyphosphorsäuren, Perfluoralkansulfonsäure (gegebenenfalls in polymerer Form), sowie gegebenenfalls Mischungen derselben. Das Verfahren wird bevorzugt in Gegenwart eines Verdünnungsmittels durchgeführt. Es kommen in Frage : Methylenchlorid, Chloroform, Dichlorethan, Tetrachlorethan, Nitrobenzol, Nitromethan. Bevorzugt ist Methylenchlorid.

Die erfindungsgemäße Reaktion ist als äußerst überraschend zu bezeichnen, da erwartet werden mußte, daß auch der Lactonring in folgender Weise reagieren würde :

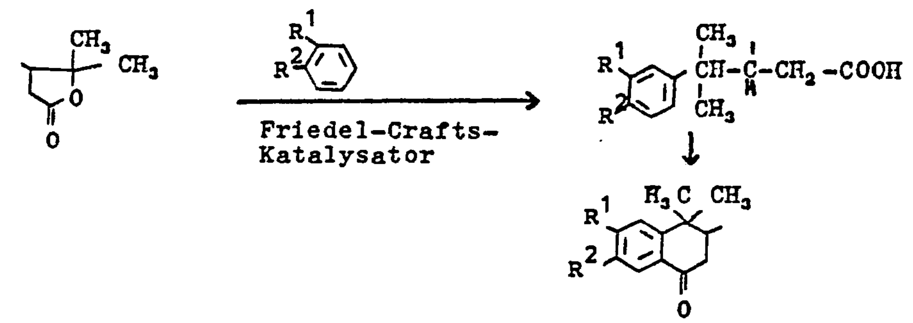


Gegebenenfalls könnte noch Ringschluß zum Tetralon erfolgen. Ringöffnungen von 5-Ring-Lactonen mit Aromaten nach Friedel-Crafts in der oben formulierten Weise sind bekannt und erfolgen unter sehr milden Bedingungen (Houben-Weyl ; Band VI, 2 Seite 812 ff). Daß eine solche Reaktion des Lactons mit den Aromaten der Formel (XXI) nicht erfolgt, ist vor allem auch deshalb überraschend, da der Friedel-Crafts-Katalysator mindestens äquimolar, besser noch im Überschuß, eingesetzt werden muß.

Um Verfahren 16. im gewünschten Sinne ablaufen zu lassen, wird folgendermaßen vorgegangen :

Man legt das Säurechlorid (XX) in einem Verdünnungsmittel vor und fügt bei Temperaturen zwischen − 10 und + 5 °C den Friedel-Crafts-Katalysator hinzu Anschließend tropft man den Aromaten, gegebenenfalls ebenfalls in einem Verdünnungsmittel gelöst, zu. Verwendet man sehr wirksame Friedel-Crafts-Katalysatoren, geschieht dies bei − 10 bis + 5 °C (z. B. bei Aluminiumchlorid, Zinntetrachlorid), bei weniger wirksamen Friedel-Crafts-Katalysatoren (z. B. Zinkchlorid, Eisenchlorid, Titantetrachlorid, Perfluoralkansulfonsäuren) tropft man den Aromaten bei Raumtemperatur zu. Anschließend rührt man bei Raumtemperatur nach ; bei weniger wirksamen Katalysatoren muß gegebenenfalls bei erhöhter Temperatur gearbeitet werden.

Die Aufarbeitung erfolgt in üblicher Weise : die Lactone können durch Umkristallisieren gereinigt werden.

Verfahren 17 kann durch folgende Reaktionsgleichung beschrieben werden :

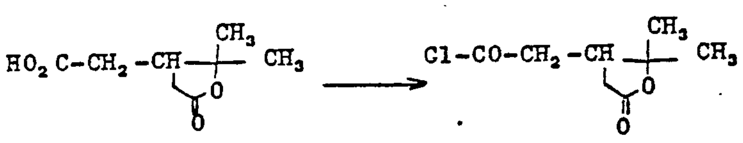

Die als Ausgangsstoff verwendete Säure der Formel (XXII) ist bekannt. Sie wird als « Terpenylsäure » bezeichnet und kann durch Oxidation von Terpentinöl erhalten werden. Das Säurechlorid der Formel (XX) ist neu. Daß eine glatte Umwandlung der Säure in das Säurechlorid gelingt, erscheint überraschend, da unter diesen Bedingungen üblicherweise auch eine Ringöffnung des Lactons erfolgt.

Verfahren 17. wird unter den Bedingungen durchgeführt, die zur Herstellung eines Säurechlorids aus einer Säure üblich sind. Bevorzugte Chlorierungsmittel sind Thionylchlorid und Phosgen. Es ist jedoch darauf zu achten, daß möglichst kurze Reaktionszeiten angewendet werden, um eine Nebenreaktion im oben erwähnten Sinne zu vermeiden. Die Aufarbeitung erfolgt in üblicher Weise. Das Säurechlorid kann durch Destillation gereinigt oder roh in Verfahren 16. eingesetzt werden.

Die aus den Styrylcyclopropancarbonsäureestern der Formel (I) erhältlichen Styrylcyclopropancarbonsäureester, soweit sie mit bei Pyrethroiden üblichen Alkoholen verestert sind, eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und

resistente Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp. Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Beispiel

Phaedon-Larven-Test

Lösungsmittel : 3 Gewichtsteile Aceton.
Emulgator : 1 Gewichtsteil Alkylarylpolyglykoläther.

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden ; 0 % bedeutet, daß keine Käfer-Larven abgetötet wurden.

Bei diesem Test zeigt z. B. die folgende Verbindung überlegene Wirksamkeit gegenüber dem Stand der Technik :

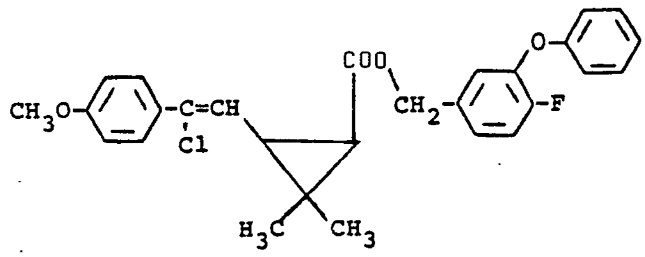

Beispiel 1 (Verfahren 3)

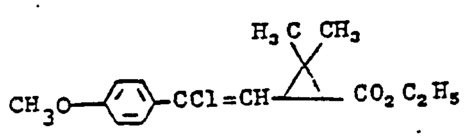

Man löst 34,5 g 5-(4'-Methoxy-phenyl)-5-chlor-3-(1'-chlor-1'-methyl)-ethyl-pent-4-en-säureethylester in 200 ml Toluol und gibt 5 g Tetrabutylammoniumbromid zu. Anschließend wird auf ca. 30 °C geheizt und innerhalb von 30 Minuten 56 g 50 %ige Kalilauge zugetropft. Die Temperatur wird dabei auf ca. 30-35 °C gehalten. Anschließend rührt man noch 2 h bei 35 °C nach, versetzt mit 200 g Eiswasser und trennt die organische Phase ab. Die Wasserphase wird noch zweimal mit je 50 ml Toluol ausgeschüttelt, die vereinigten organischen Phasen mit Wasser, dem etwas 1 n HCl zugesetzt wird, neutral gewaschen, mit $Na_2SO_4$ getrocknet und einrotiert. Durch Hochvakuumdestillation werden 25 g roher 2,2-Dimethyl-3-[2'-chlor-2'-(4'-methoxy-phenyl)-vinyl]-cyclopropan-1-carbonsäureethylester erhalten (Isomerengemisch ; eins der vier Isomeren entsteht ganz überwiegend), der durch eine zweite Destillation noch weiter gereinigt werden kann. Siedepunkt : 162-168 °C/0,08 mbar.

Analog werden erhalten (Verfahren 3) : 2,2-Dimethyl-3-[2'-chlor-2'-(4'-trifluormethoxy-phenyl)-vinyl]-cyclopropan-1-carbonsäureethylester 2,2-Dimethyl-3-[2'-chlor-2'-(3',4'-methylendioxy-phenyl)-vinyl]-cyclopropan-1-carbonsäureethylester.

Beispiel 2 (Verfahren 2)

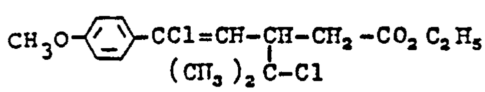

Man löst 141,0 g 4,4-Dimethyl-3-[2'-chlor-2'-(4'-methoxyphenyl)-vinyl]-γ-butyrolacton in 500 ml Toluol. Dann gibt man 125 ml Thionylchlorid zu und erhitzt 6 h auf 80 °C. Anschließend werden erneut 125 ml Thionylchlorid zugegeben und nochmals auf 80 °C erhitzt. Anschließend wird das überschüssige Thionylchlorid und etwas Toluol unter Wasserausschluß bei Normaldruck abdestilliert (ca. 250 ml). Nach dem Abkühlen werden unter Kühlung innerhalb von 3 h 400 ml Ethanol, das mit Chlorwasserstoff gesättigt ist, bei 20 °C zugetropft. Man rührt noch 3 h nach und läßt über Nacht stehen. Die Lösungsmittel werden unter vermindertem Druck (Wasserstrahlvakuum) abdestilliert. Der zurückbleibende rohe 5-(4'-Methoxy-phenyl)-5-chlor-3-(1'-chlor-1'-methyl)-ethyl-pent-4-ensäureethylester wird direkt nach Verfahren 3 weiter umgesetzt.

Analog werden erhalten (Verfahren 2) : 5-(4'-Trifluormethoxyphenyl)-5-chlor-3-(1'-chlor-1'-methyl)-ethyl-pent-4-en-säureethylester, 5-(3,4'-Methylendioxy-phenyl)-5-chlor-3-(1'-chlor-1'-methyl)-ethyl-pent-4-en-säureethylester.

Beispiel 3 (Verfahren 2)

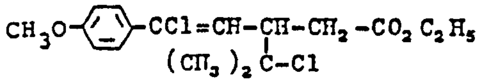

Man mischt in einem 0,7 1-Autoklaven 134 g 4,4-Dimethyl-3-[2'-chlor-2'-(4'-methoxyphenyl)-vinyl]-γ-butyrolacton mit 120 g Thionylchlorid. Anschließend werden 50 g Ethanol zugepumpt. Dann heizt man den Autoklaven noch 1 h auf 80 °C, läßt abkühlen und entspannt. Überschüssiges Thionylchlorid bzw. Schwefelsäureethylester werden durch Destillation im Wasserstrahlvakuum entfernt. Der Rückstand besteht im wesentlichen aus 5-(4'-Methoxy-phenyl)-5-chlor-3-(1'-chlor-1'-methyl)-ethyl-pent-4-en-säure-ethylester und wird direkt nach Verfahren 3 weiter umgesetzt.

Beispiel 4 (Verfahren 6a)

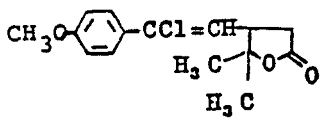

Man löst 52,4 g 4,4-Dimethyl-3-(4'-methoxy-phenacyl)-γbutyrolacton in 300 ml Toluol, dosiert 87 g Phosphorpentachlorid hinzu und rührt bei Raumtemperatur solange nach, bis alles in Lösung gegangen ist (ca. 8 h). Dann tropft man bei 20-50 °C 500 ml Wasser hinzu und rührt 4 h nach. Anschließend wird die Toluolphase abgetrennt, mit Natriumsulfat getrocknet und im Wasserstrahlvakuum eingeeingt. Es bleibt 4,4'-Dimethyl-3-[2'-chlor-2'-(4'-methoxy-phenyl)-vinyl]-γ-butyrolacton (E- und Z-Isomeres) zurück.

Beispiel 5 (Verfahren 6b)

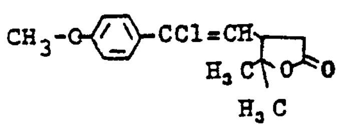

40 g rohes 4,4-Dimethyl-3-[2'-chlor-2'-(4'-methoxyphenyl)-vinyl]-2-ethoxycarbonyl-γ-butyrolacton werden in 400 ml 25 %iger Schwefelsäure suspendiert und bei Normaldruck so lange Wasser und Ethanol abdestilliert, bis die Innentemperatur 115-120 °C erreicht hat. Dan erhitzt man noch 10 h bei dieser Temperatur (ohne weiteres Abdestillieren), läßt abkühlen und extrahiert mit Methylenchlorid. Nach Trocknen mit Natriumsulfat und Abziehen des Lösungsmittels hinterbleibt ein dunkler Rückstand, der nach kurzem Stehen teilweise kristallisiert. Es handelt sich aufgrund des NMR-Spektrums um 4,4-Dimethyl-3-[2'-chlor-2'-(4'-methoxy-phenyl)-vinyl]-γ-butyrolacton (E- und Z-Isomeres).

Analog Verfahren 6b werden erhalten: 4,4-Dimethyl-3-[2'-chlor-2'-(4'-trifluormethoxy-phenyl)-vinyl]-γ-butyrolacton, 4,4-Dimethyl-3-[2'-chlor-2'-(3',4'-methylendioxyphenyl)-vinyl]-γ-butyrolacton.

Beispiel 6 (Verfahren 9)

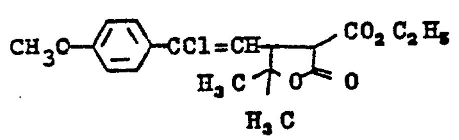

Man löst 3,5 g Natrium in 350 ml Ethanol und tropft bei Raumtemperatur 24 g Malonsäurediethylester hinzu.

Dann werden bei 30-35 °C 35,8 g 2,2-Dimethyl-3-[2'-chlor-2'-(4'-methoxyphenyl)-vinyl]-oxiran langsam zugetropft. Nach beendetem Zutropfen hält man noch 4 h auf 35 °C und läßt dann abkühlen. (An dieser Stelle kann sich gegebenenfalls direkt Verfahren 6b anschließen).

Man destilliert im Vakuum ein Teil des Ethanols ab, versetzt mit Eiswasser und stellt sauer. Dann wird mit Methylenchlorid extrahiert, die organische Phase mit Natriumsulfat getrocknet und das Lösungsmittel abdestilliert. Der Rückstand wiegt 39 g und besteht — wie durch IR-Spektrum, NMR-Spektrum und Massenspektrum belegt — hauptsächlich aus 4,4-Dimethyl-3-[2'-chlor-2'-(4'-methoxyphenyl)-vinyl]-2-ethoxycarbonyl-γ-butyrolacton.

Analog Verfahren 9 werden erhalten: 4,4-Dimethyl-3-[2'-chlor-2'-(4'-trifluormethoxyphenyl)-vinyl]-2-ethoxy-carbonyl-γ-butyrolacton, 4,4-Dimethyl-3-[2'-chlor-2'-(3',4'-methylendioxyphenyl)-vinyl]-2-ethoxy-carbonyl-γ-butyrolacton.

Beispiel 7 (Verfahren 12)

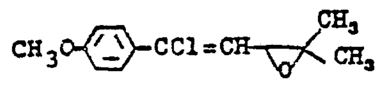

89 g (0,4 Mol) 1-(4'-Methoxyphenyl)-1-chlor-4,4-dimethyl-1,3-butadien werden in 640 ml Methylenchlorid gelöst und 160 g pulverisiertes wasserfreies Natriumcarbonat zugegeben. Dann dosiert man bei Raumtemperatur 69 g (0,4 Mol) m-Chlor-perbenzoesäure hinzu und rührt 2 h bei Raumtemperatur nach. Anschließend wird abgesaugt, mit $CH_2Cl_2$ nachgewaschen und die Methylenchloridlösung zweimal mit viel Natriumbicarbonatlösung geschüttelt. Nach Neutralwaschen mit Wasser, wird die Methylenchloridphase mit $Na_2SO_4$ getrocknet und einrotiert. Der hellgelbe flüssige Rückstand ($n_D^{20}$ = 1,481) enthält nach GC drei Komponenten mit der Masse m/e = 238 (GC/MS). Der Gehalt an den gewünschten stereoisomeren Epoxiden beträgt 91 %. Das Produkt (80 g) wird direkt in die nächste Stufe eingesetzt. Analog Verfahren 12 werden erhalten : 2,2-Dimethyl-3-[2'-chlor-2'-(4'-trifluormethoxyphenyl)-vinyl] oxiran, 2,2-Dimethyl-3-[2'-chlor-2'-(3',4'-methylendioxy-phenyl)-vinyl] oxiran.

Beispiel 8 (Verfahren 14a)

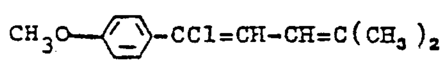

Man löst 17 g Kaliumhydroxid in 125 ml Wasser und 125 ml Methanol und gibt 150 g 4-Methoxyacetophenon hinzu. Bei 50 °C werden 80 g Isobutyraldehyd zugetropft (innerhalb von 1,5 Stunden) Nach 3, 5 Stunden wird auf Raumtemperatur gekühlt und mit Essigsäure neutralisiert (ca. 20 ml). Das dimere Kondensationsprodukt wird abgesaugt, mit Methanol gewaschen und an der Luft getrocknet. Ausbeute 175 g. Der Feststoff wird mit 5 g Natriumacetat vermischt und im Wasserstrahlvakuum destilliert. Man erhält 165 g eines bei Raumtemperatur erstarrenden gelben öls, das ein Gemisch aus 2-Methyl-5-oxo-5-(4'-methoxy-phenyl)-2-penten und 2-Methyl-5-oxo-5-(4'-methoxy-phenyl)-3-penten darstellt. Siedepunkt des Isomerengemisches : Kp = 158-165 °C/17 mbar.

Man löst 102 g (0,5 Mol) des oben erhaltenen Isomerengemisches in 500 ml Toluol und dosiert 104 g Phosphorpentachlorid bei 0 °C langsam hinzu. Man rührt solange bei 0-10 °C, bis alles $PCl_5$ in Lösung gegangen ist, gibt 5 g Tetrabutylammoniumbromid zu und tropft unter guter Kühlung bei 0-10 °C 224 g (2 Mol) 50 %ige Kalilauge zu. Anschließend trennt man die Toluolphase sofort ab, wäscht neutral und rotiert ein. Anschließend wird das rohe Dien überdestilliert (Kp = 125-165 °C/0,8 mm) und anschließend durch eine zweite Destillation gereinigt. Man erhält 75 g 1-(4'-Methoxy-phenyl)-1-chlor-4,4-dimethyl-1,3-butadien vom Siedepunkt 112-119 °C/0,2 mm.

Beispiel 9 (Verfahren 14b)

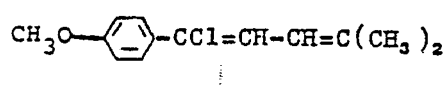

4,6 g (0,2 Mol) Natrium werden portionsweise in 100 ml trockenem Ethanol gelöst. Wenn sich alles Natrium aufgelöst hat, werden 100 ml wasserfreies Tetrahydrofuran zugesetzt und bei 0 °C unter Rühren 58,4 g (0,2 Mol) 4-Methoxy-α-chlor-benzyl-phosphonsäurediethylester, gelöst in 50 ml wasserfreiem THF, zugetropft. Nachdem noch 1 Stunde bei 0-10 °C nachgerührt wurde, werden 16,8 g (0,2 Mol) β,β-Dimethylacrolein, gelöst in 30 ml wasserfreiem Tetrahydrofuran, unter Rühren zugetropft. Anschließend wird noch 12 Stunden bei Raumtemperatur nachgerührt. Die Reaktionsmischung wird dann mit 600 ml Wasser versetzt und zweimal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 25 g 1-(4'-Methoxyphenyl)-1-chlor-4,4-dimethyl-1,3-butadien (Isomerengemisch) als gelbes, nach einiger Zeit teilweise kristallisierendes Öl, mit dem Siedepunkt 130-145 °C/2 Torr.

Analog erhält man (Verfahren 14b) :

Beispiel 10

1-(3',4'-Methylendioxy-phenyl)-1-chlor-4,4-dimethyl-1,3-butadien.

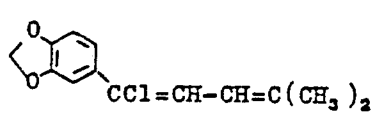

Die als Vorprodukte benötigten α-Hydroxy-4-methoxy-benzyl-phosphonsäureester der Formel

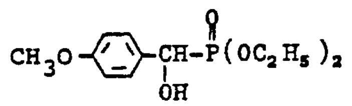

können wie folgt hergestellt werden :

Beispiel 11

In ein Gemisch aus 20,7 g (0,15 Mol) Diethylphosphit und 1,09 g (0,010 9 Mol) Triethylamin werden innerhalb einer Stunde unter Wasserkühlung bei 50-70 °C 20,4 g (0,150 Mol) 4-Methoxybenzaldehyd eindosiert. Der Reaktionsansatz wird 1 Stunde bei 70 °C nachgerührt. Nach dem Abkühlen wird der Ansatz mit 40 g Toluol aufgenommen und mehrfach mit verdünnter Salzsäure und kaltem Wasser nachgewaschen. Die organische Schicht wird abgetrennt und im Vakuum vom Lösungsmittel befreit. Die Ausbeute beträgt 37 g an 4-Methoxy-α-hydroxy-benzyl-phosphonsäure-diethylester.

Beispiel 12

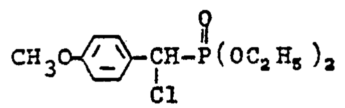

Einem Gemisch aus 20,2 g (0,072 5 Mol) 4-Methoxy-α-hydroxy-benzyl-phosphonsäure-diethylester, 65 g Dichlormethan und 5,8 g (0,072 5 Mol) Pyridin werden bei 20-40 °C unter leichter Wasserkühlung innerhalb von etwa 1 Stunde 9,2 g (0,076 8 Mol) Thionylchlorid hinzugesetzt. Das Reaktionsgemisch wird dann 3 Stunden unter Rückfluß erhitzt und 12 Stunden ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 6 Torr und 45 °C eingeengt. Man erhält 21 g (97,7 % der Theorie) 4-Methoxy-α-chloro-benzyl-phosphonsäure-diethylester als gelbes viskoses Öl mit einer Reinheit von 98,6 % (Gaschromatogramm) und einem Brechungsindex von $n_D^{24}$ : 1.511 8.

Beispiel 13 (Verfahren 14c)

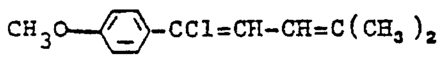

Zu einer Suspension von 22,7 g (0,052 5 Mol) Triphenylisopropyl-phosphoniumjodid in 100 ml Tetrahydrofuran werden bei 0 °C 0,055 Mol n-Butyl-lithium in 30 ml Hexan getropft. Es wird 1 Stunde bei 0 °C nachgerührt. Dann wird in einem Guß eine Lösung von 10 g (0,05 Mol) β-(4-Methoxy-phenyl)-β-chlor-acrolein in 100 ml THF zugegeben und dann 10 Stunden bei 20 °C nachgerührt. Das Reaktionsgemisch wird darauf eingeengt, mit 100 ml Wasser versetzt und zweimal mit je 50 ml Ether extrahiert. Die vereinigten Etherextrakte werden über $Na_2SO_4$ getrocknet und eingeengt. Es verbleit ein Rückstand, der bei Zugabe von ca. 50 ml Petrolether kristallisiert. Die Kristalle werden abgesaugt und noch einmal aus Petrolether umkristallisiert. Man erhält 8 g 1-(4'-Methoxyphenyl)-1-chlor-4,4-dimethyl-1,3-butadien in Form von hellgelben Kristallen.

Beispiel 14 (Verfahren 14d)

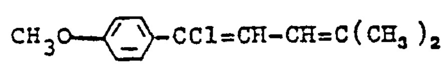

Zu einer Lösung von 0,4 Mol Isopropylmagnesiumchlorid in 40 ml Isopropylchlorid (hergestellt nach J. Houben et. al. Chem. Ber. *69* (1936), 1766) werden bei 20 °C 39,3 g (0,2 Mol) β-Chlor-β-(4-Methoxyphenyl)-acrolein in 200 ml Tetrahydrofuran getropft. Es wird 12 Stunden nachgerührt. Dann wird die Reaktionsmischung unter Rühren in eine Mischung von 500 g Eis und 100 ml $H_2SO_4$ gegossen. Es wird zweimal mit je 100 ml Ether extrahiert. Die vereinigten Etherextrakte werden über Natriumsulfat getrocknet und bei 50 °C am Rotationsverdampfer eingeengt.

Der Rückstand wird in 50 ml Eisessig aufgenommen. Zu dieser Lösung werden 2 ml konz. $H_2SO_4$ in der Weise getropft, daß die Temperatur 45 °C nicht übersteigt. Es wird 4 h bei 20 °C nachgerührt. Dann wird die Reaktionsmischung in Wasser gegeben. Es wird zweimal mit je 100 ml Ether extrahiert. Die vereinigten Etherextrakte werden über $Na_2SO_4$ getrocknet, eingeengt und destilliert. Man erhält 22 g 1-(4'-Methoxyphenyl)-1-chlor-4,4-dimethyl-1,3-butadien in Form eines gelben Öles, das beim Abkühlen teilweise kristallisiert. Kp. = 140-145 °C/4 mm.

Beispiel 15 (Verfahren 15)

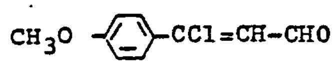

750 g (5 Mol) 4-Methoxyacetophenon werden in 2,5 l Dimethylformamid gelöst, bei 40-45 °C unter leichter Kühlung innerhalb von 2 Stunden tropfenweise mit 1 570 g Phosphoroxichlorid versetzt, 1 Stunde verrührt, auf 25 l Eiswasser ausgetragen und durch Zutropfen von ca. 2,1 l konzentrierter Natronlauge ein pH-Wert von 5 eingestellt. Die Temperatur wird während der Neutralisation durch Einwerfen von Eis unter 25 °C gehalten. Anschließend wird noch 1 Stunde bei pH 5 und bei einer Temperatur unter 25 °C nachgerührt. Der helle kristalline Niederschlag wird abgesaugt, gründlich mit Wasser gewaschen und bei 50 °C im Vakuum getrocknet. Ausbeute 730 g.

Beispiel 16 (Verfahren 16)

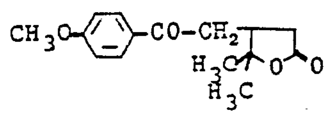

Man legt 80 g Aluminiumchlorid in 300 ml Methylenchlorid vor und tropft 59 g 4,4-Dimethyl-3-chlorcarbonylmethyl-γ-butyrolacton in 150 ml Methylenchlorid, gelöst bei 0-5 °C, zu. Anschließend tropft man 32,4 g Anisol in 50 ml Methylenchlorid gelöst ebenfalls bei 0-5 °C zu. Dann läßt man auf Raumtemperatur kommen und rührt noch 7 h bei Raumtemperatur nach. Nach Eingießen des Ansatzes in Eiswasser wird die organische Phase abgetrennt und neutral gewaschen. Nach Trocknen und Abdestillieren des Lösungsmittels sowie des überschüssigen Anisols erhält man 78 g rohes 4,4-Dimethyl-3-(4'-methoxy)-phenacyl-γ-butyrolacton. Smp. 83 °C.

Beispiel 17 (Verfahren 16)

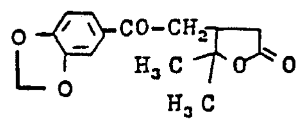

Man legt 107 g Aluminiumchlorid in 400 ml Methylenchlorid vor und tropft 76 g 4,4-Dimethyl-3-chlorcarbonylmethyl-γ-butyrolacton in 100 Methylenchlorid gelöst bei − 5 bis 0 °C zu. Anschließend tropft man 55 g Benzodioxol in 50 ml Methylenchlorid bei 0-10 °C zu. Dann läßt man auf Raumtemperatur kommen und rührt noch 4 h nach. Nach Eingießen des Ansatzes in 1 Liter Eiswasser wird die organische Phase abgetrennt und neutral gewaschen. Nach Trocknen und Abdestillieren des Lösungsmittels erhält man 4,4-Dimethyl-3-(3',4'-methylendioxy-phenacyl)-γ-butyrolacton.

Beispiel 18 (Verfahren 18)

Man vermischt 172 g 4,4-Dimethyl-3-carboxymethyl-γ-butyrolacton mit 600 ml Thionylchlorid und erhitzt 1 h auf 80 °C. Dann destilliert man das überschüssige Thionylchlorid bei Normaldruck ab, die letzten Reste im Wasserstrahlvakuum. Der Rückstand besteht aus 4,4-Dimethyl-3-chlorcarbonylmethyl-γ-butyrolacton ($n_D^{20}$ = 1,484) und kann direkt für Verfahren 16 verwendet werden. Es kann jedoch auch noch durch Destillation weiter gereinigt werden: Siedepunkt: 130-140 °C/0,3 mbar (bei größeren Mengen muß am Dünnschichter destilliert werden). Das reine Säurechlorid ist fest. Smp. 64 °C.

Beispiel 19 (nicht erfindungsgemäß)

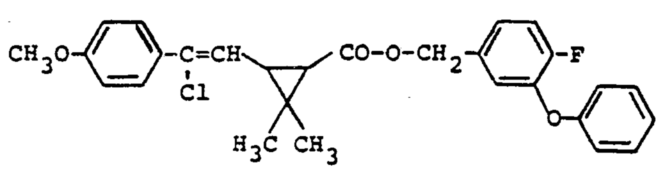

4,4 g (0,02 Mol) 3-Phenoxy-4-fluoro-benzylalkohol und 7,1 g (0,02 Mol) 2,2-Dimethyl-3-(2-chloro-2-(4-methoxy-phenyl)-vinyl)-cyclopropancarbonsäurechlorid werden in 100 ml wasserfreiem Toluol gelöst und bei 20-25 °C 2,5 g Pyridin, gelöst in 50 ml wasserfreiem Toluol, unter Rühren zugetropft. Anschließend wird weitere 3 Stunden bei 25-35 °C gerührt. Das Reaktionsgemisch wird in 150 ml Wasser, dem 10 ml konzentrierte Salzsäure zugesetzt werden, gegossen, die organische Phase abgetrennt und

nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60 °C/1 Torr Badtemperatur entfernt. Man erhält 8,1 g (84,5 % der Theorie) 2,2-Dimethyl-3-(2-chloro-2-(4-trifluormethoxy-phenyl)-vinyl)-cyclopropancarbonsäure-(4-fluoro-3-phenoxy-benzyl)-ester.

**Ansprüche**

1. Verbindungen der allgemeinen Formel (II)

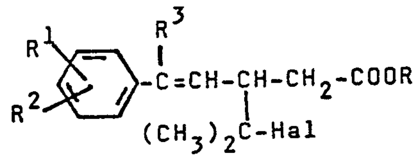

(II)

in welcher
$R^1$ für Alkoxy, Alkylthio, die gegebenenfalls durch Halogen substituiert sind, steht,
$R^2$ für Wasserstoff, Alkoxy oder gemeinsam mit $R^1$ für gegebenenfalls halogensubstituiertes Alkylendioxy steht und
$R^3$ für Wasserstoff oder Chlor steht, und
R für $C_{1-4}$-Alkyl steht,
Hal für Chlor oder Brom steht.

2. Verfahren zur Herstellung von Verbindungen der allgemeinen Formel (II)

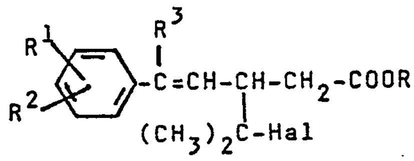

(II)

in welcher
$R^1$ für Alkoxy, Alkylthio, die gegebenenfalls durch Halogen substituiert sind, steht,
$R^2$ für Wasserstoff, Alkoxy oder gemeinsam mit $R^1$ für gegebenenfalls halogensubstituiertes Alkylendioxy steht und
$R^3$ für Wasserstoff oder Chlor steht, und
R für $C_{1-4}$-Alkyl steht und
Hal für Chlor oder Brom steht, dadurch gekennzeichnet, daß Verbindungen der allgemeinen Formel (III)

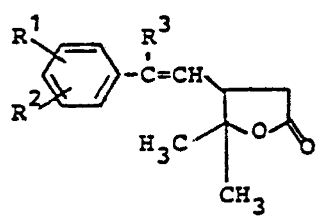

(III)

in welcher
$R^1$, $R^2$ und $R^3$ die oben angegebene Bedeutung haben, mit einem Halogenierungsmittel sowie gleichzeitig oder nacheinander mit einem Alkohol der Formel (IV)

$$R—OH \qquad (IV)$$

in welcher
R die oben angegebene Bedeutung hat, gegebenenfalls in Gegenwart eines Verdünnungsmittels und gegebenenfalls in Gegenwart von gasförmigem Halogenwasserstoff, umgesetzt werden.

3. Verwendung der Verbindungen der Formel (II) zur Herstellung von Styryl-cyclopropan-1-carbonsäureester der allgemeinen Formel (I)

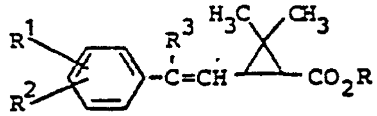

(I)

in welcher
R für $C_{1-4}$-Alkyl steht,
$R^1$ für Alkoxy, Alkylthio, die gegebenenfalls durch Halogen substituiert sind, steht,
$R^2$ für Wasserstoff, Alkoxy oder gemeinsam mit $R^1$ für gegebenenfalls halogensubstituiertes Alkylendioxy steht und
$R^3$ für Wasserstoff oder Chlor steht, dadurch gekennzeichnet, daß man Verbindungen der allgemeinen Formel (II)

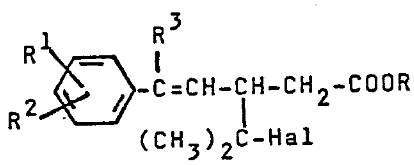

(II)

in welcher
R, $R^1$, $R^2$, $R^3$ die oben angegebene Bedeutung besitzen und
Hal für Chlor oder Brom steht, mit einer gegebenenfalls wässrigen Base gegebenenfalls in Gegenwart eines Verdünnungsmittels in Gegenwart eines Phasentransferkatalysators umsetzt.

4. Verfahren gemäß Anspruch 3, dadurch gekennzeichnet, daß nicht zu starke Basen verwendet werden und in Gegenwart eines Phasentransferkatalysators gearbeitet wird.

**Claims**

1. Compounds of the general formula (II)

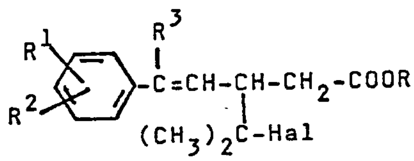

(II)

in which
$R^1$ represents alkoxy or alkylthio which are optionally substituted by halogen,
$R^2$ represents hydrogen or alkoxy or together with $R^1$ represents optionally halogen-substituted alkylenedioxy and
$R^3$ represents hydrogen or chlorine, and
R represents $C_{1-4}$-alkyl, and
Hal represents chlorine or bromine.

2. Process for the preparation of compounds of the general formula (II)

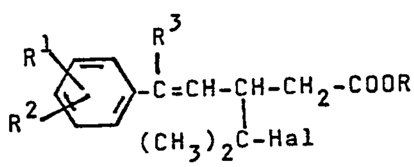

(II)

in which
$R^1$ represents alkoxy or alkylthio which are optionally substituted by halogen,
$R^2$ represents hydrogen or alkoxy or together with $R^1$ represents optionally halogen-substituted alkylenedioxy and
$R^3$ represents hydrogen or chlorine, and
R represents $C_{1-4}$-alkyl, and
Hal represents chlorine or bromine, characterised in that compounds of the general formula (III)

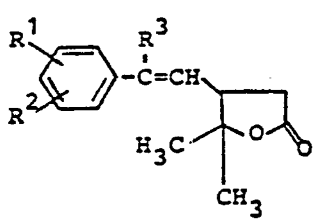

(III)

in which

$R^1$, $R^2$ and $R^3$ have the meaning indicated above, are reacted with a halogenating agent and at the same time or successively, with an alcohol of the formula (IV)

R—OH (IV)

in which

R has the meaning indicated above, if appropriate in the presence of a diluent and if appropriate in the presence of a gaseous hydrogen halide.

3. Use of the compounds of the formula (II) for the preparation of styryl-cyclopropane-1-carboxylic acid esters of the general formula (I)

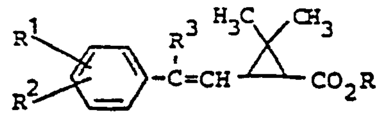

(I)

in which

R represents $C_{1-4}$-alkyl,

$R^1$ represents alkoxy or alkylthio which are optionally substituted by halogen,

$R^2$ represents hydrogen or alkoxy or together with $R^1$ represents optionally halogen-substituted alkylenedioxy and

$R^3$ represents hydrogen or chlorine, characterised in that compounds of the general formula (II)

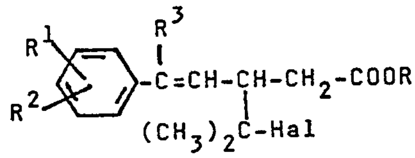

(II)

in which

R, $R^1$, $R^2$ and $R^3$ have the meaning indicated above and

Hal represents chlorine or bromine, are reacted with an optionally aqueous base, if appropriate in the presence of a diluent and in the presence of a phase transfer catalyst.

4. Process according to Claim 3, characterised in that bases which are not too strong are used and the reaction is carried out in the presence of a phase transfer catalyst.

**Revendications**

1. Composés de formule générale (II)

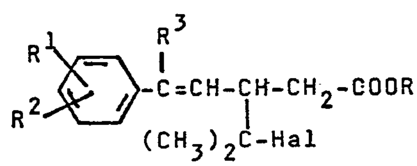

(II)

dans laquelle

$R^1$ représente un groupe alcoxy ou alkylthio éventuellement substitué par un halogène,

$R^2$ représente l'hydrogène ou un groupe alcoxy ou bien, pris avec $R^1$, un groupe alkylènedioxy éventuellement halogénosubstitué, et

$R^3$ représente l'hydrogène ou le chlore et

R représente un reste alkyle en $C_1$-$C_4$ et

Hal représente le chlore ou le brome.

2. Procédé pour la fabrication de composés de formule générale (II)

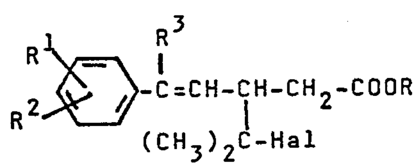

(II)

dans laquelle

$R^1$ représente un groupe alcoxy ou alkylthio éventuellement substitué par un halogène,

$R^2$ représente l'hydrogène ou un groupe alcoxy ou bien, pris avec $R^1$, un groupe alkylènedioxy éventuellement halogénosubstitué, et

$R^3$ représente l'hydrogène ou le chlore et

R représente un reste alkyle en $C_1$-$C_4$ et

Hal représente le chlore ou le brome, caractérisé en ce que l'on fait réagir des composés de formule générale (III)

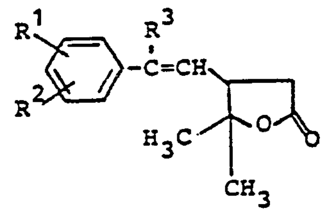

(III)

dans laquelle

$R^1$, $R^2$ et $R^3$ ont la signification indiquée ci-dessus, avec un agent d'halogénation et simultanément, ou l'un après l'autre, avec un alcool de formule (IV)

R—OH (IV)

dans laquelle

R a la signification indiquée ci-dessus, éventuellement en présence d'un agent diluant et éventuellement en présence d'un halogénure d'hydrogène gazeux.

3. Utilisation des composés de formule (II) pour la fabrication d'esters d'acides styrylcyclopropane-1-carboxyliques de formule générale (I)

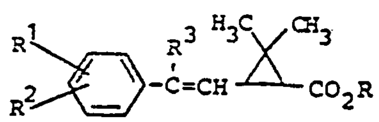

(I)

dans laquelle

R représente un reste alkyle en $C_1$-$C_4$,

$R^1$ représente un groupe alcoxy ou alkylthio éventuellement substitué par un halogène,

$R^2$ représente l'hydrogène ou un groupe alcoxy ou bien, pris avec $R^1$, un groupe alkylènedioxy éventuellement halogénosubstitué, et

$R^3$ représente l'hydrogène ou le chlore, caractérisé en ce que l'on fait réagir des composés de formule générale (II)

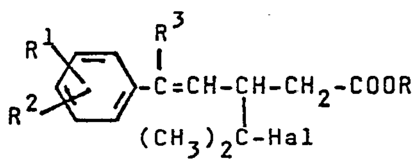

(II)

dans laquelle

R, $R^1$, $R^2$ et $R^3$ ont la signification indiquée ci-dessus et

Hal représente le chlore ou le brome, avec une base éventuellement aqueuse, en présence d'un catalyseur de transfert de phase éventuellement, en présence d'un agent diluant.

4. Procédé selon la revendication 3, caractérisé en ce que l'on utilise des bases pas trop fortes et on opère en présence d'un catalyseur de transfert de phase.